# EUROPÄISCHE PATENTSCHRIFT

(11) **EP 1 349 844 B1**
(45) Veröffentlichungstag und Bekanntmachung des Hinweises auf die Patenterteilung: **11.08.2004**
(21) Anmeldenummer: 01984809.2
(22) Anmeldetag: 07.12.2001
(51) Int. Cl.: C07D 307/08

(54) **VERFAHREN ZUR HYDRIERUNG VON MALEINSÄUREANHYDRID UND VERWANDTEN VERBINDUNGEN IN ZWEI HINTEREINANDER GESCHALTETEN REAKTIONSZONEN**
METHOD FOR THE HYDROGENATION OF MALEIC ANHYDRIDE AND RELATED COMPOUNDS IN TWO SERIAL REACTION ZONES
PROCEDE D'HYDROGENATION D'ANHYDRIDE DE L'ACIDE MALEIQUE ET DE COMPOSES VOISINS DANS DEUX ZONES DE REACTION EN SERIE

(30) Priorität: 11.12.2000 DE 10061557
(43) Veröffentlichungstag der Anmeldung: 08.10.2003
(73) Patentinhaber: BASF AKTIENGESELLSCHAFT, 67056 Ludwigshafen (DE)
(72) Erfinder: BORCHERT, Holger, 67591 Offstein (DE); SCHLITTER, Stephan, 67117 Limburgerhof (DE); FISCHER, Rolf-Hartmuth, 69121 Heidelberg (DE); RÖSCH, Markus, 55276 Oppenheim (DE); STEIN, Frank, 67098 Bad Dürkheim (DE); RAHN, Ralf-Thomas, 68167 Mannheim (DE); WECK, Alexander, 67251 Freinsheim (DE); KAIBEL, Gerd, 68623 Lampertheim (DE)
(74) Vertreter: Isenbruck, Günter, Dr.
(86) Internationale Anmeldenummer: PCT/EP2001/014393
(87) Internationale Veröffentlichungsnummer: WO 2002/048129

(56) Entgegenhaltungen:
- WO-A-99/35136
- US-A- 5 072 009
- US-A- 5 149 836

## Beschreibung

Erfindung betrifft ein Verfahren zur Herstellung von gegebenenfalls alkylsubstituiertem γ-Butyrolacton und Tetrahydrofuran durch katalytische Hydrierung in der Gasphase von Substraten, die ausgewählt sind aus der Gruppe bestehend aus Maleinsäure und Bernsteinsäure und Derivaten dieser Säuren. Darunter werden im Rahmen der vorliegenden Erfindung Ester und Anhydride verstanden, wobei diese, ebenso wie die Säuren, einen oder mehrere Alkylsubstituenten aufweisen können. Mit dem Verfahren können hohe Ausbeuten erzielt werden und das Verhältnis der beiden Produkte zueinander mit hoher Variabilität eingestellt werden kann. Das erfindungsgemäße Verfahren wird in zwei hintereinandergeschalteten Reaktionszonen durchgeführt.

Die Herstellung von γ-Butyrolacton (GBL) und Tetrahydrofuran (THF) durch Gasphasenhydrierung von Maleinsäureanhydrid (MSA) ist eine seit vielen Jahren bekannte Reaktion. Zur Durchführung dieser katalytischen Reaktion sind in der Literatur zahlreiche Katalysatorsysteme beschrieben. Diese sind zum großen Teil häufig Cr-haltig. Je nach Zusammensetzung des Katalysators und den gewählten Reaktionsparametern werden mit derartigen Katalysatoren unterschiedliche Produktverteilungen erreicht.

Mögliche weitere Edukte zur Herstellung von GBL und THF sind neben MSA die Maleinsäure selbst, Bernsteinsäure und deren Anhydrid sowie Ester dieser Säuren. Sollen GBL und THF hergestellt werden, die Alkylsubstituenten aufweisen, so bietet es sich an, von den vorstehend genannten Säuren, Estern und Anhydriden auch die entsprechend alkylsubstituierten Spezies zu verwenden.

In der US 3,065,243 ist ein Verfahren offenbart, bei dem Kupferchromit als Katalysator dient. Laut Beschreibung und Beispielen entstehen bei dieser Reaktionsführung noch beträchtliche Mengen an Bernsteinsäureanhydrid (BSA), das im Kreis gefahren werden muß. Wie bekannt ist, treten dabei häufig verfahrenstechnische Probleme aufgrund der Kristallisation des BSA oder auch daraus entstehender Bernsteinsäure mit anschließender Verstopfung von Rohrleitungen auf.

Die Offenbarung von weiteren Kupferchromit-Katalysatoren zur Hydrierung von MSA finden sich zum Beispiel in den Druckschriften US 3,580,930, US 4,006,165, der EP-A 638 565 sowie der WO 99/38856. Laut Offenbarung lassen sich mit den dort beschriebenen Katalysatoren hohe Ausbeuten an GBL erzielen. THF wird jeweils nur in Spuren gebildet. Oftmals ist es jedoch so, daß höhere Mengen an THF aus mehreren Gründen erwünscht sind.

Ein Verfahren, das dies gestattet, wird in der US 5,072,009 offenbart. Die gemäß diesem Patent verwendeten Katalysatoren entsprechen der allgemeinen Formel CuₗZn_{b}Al_{c}M_{d}Oₓ, in der M mindestens ein Element ist, das ausgewählt ist aus der Gruppe bestehend aus den Gruppen IIA und IIIA, VA, VIII, Ag, Au, den Gruppen IIIB bis VIIB sowie Lanthaniden und Aktinoiden des Periodensystems der Elemente; b ist eine Zahl zwischen 0,001 und 500, c eine Zahl zwischen 0,001 und 500 und d eine Zahl von 0 bis < 200 und x entspricht der Anzahl an Sauerstoffatomen, die nach den Valenzkriterien notwendig sind. Obwohl ausgesagt wird, daß die Katalysatoren entsprechend dieser Patentschrift kein Chrom enthalten müssen, werden in allen Beispielen chromhaltige Katalysatoren beschrieben. Nach diesen Beispielen wird eine maximale THF-Ausbeute von 96% erhalten, die Hydrierung wird bei Drücken von 20 bis 40 bar durchgeführt.

Prinzipiell nachteilig an allen oben beschriebenen Katalysatorsystemen ist die Anwesenheit von Chromoxid, dessen Verwendung aufgrund der akuten Toxizität vermieden werden sollte. Auch derartige Cr-freie Katalysatorsysteme zur Herstellung von GBL durch Hydrierung von MSA sind im Stand der Technik beschrieben. Beispiele für derartige Katalysatorsysteme finden sich in den Druckschriften WO 99/35139 (Cu-Zn-Oxid), WO 95/22539 (Cu-Zn-Zr) sowie der US 5,122,495 (Cu-Zn-Al-Oxid). Alle diese Katalysatorsysteme ermöglichen hohe Ausbeuten an GBL, bis zu 98%, dabei wird die Bildung von THF jedoch nicht oder nur in Spuren beobachtet. Zwar läßt sich dessen Bildung, wie bekannt ist, durch eine Erhöhung der Reaktionstemperatur oder längere Verweilzeiten im Reaktor begünstigen, gleichzeitig steigt jedoch auch der Anteil unerwünschter Nebenprodukte, beispielsweise Butanol, Butan, Ethanol oder Ethan.

Ein ausschließlich aus Cu- und Al-Oxiden aufgebauter Katalysator für die MSA-Gasphasenhydrierung zu GBL wird in der WO 97/24346 offenbart. Auch hier finden sich die gleichen Nachteile wie bei den im vorstehenden Absatz beschriebenen Druckschriften, nämlich nur untergeordnete bzw. spurenweise Bildung von THF.

Die Verwendung eines Katalysators mit prinzipiell gleicher Zusammensetzung wie in der WO 97/24346 beschrieben, nämlich basierend auf Cu-Al-Oxiden, wird auch in der JP 2 233 631 offenbart. Das Ziel dieser Erfindung liegt dabei darin, die Hydrierung von MSA so durchzuführen, daß als Hauptprodukte THF und 1,4-Butandiol neben nur geringen oder gar keinen Mengen GBL entstehen. Dieses wird dann durch die Verwendung der auf gemischten Cu-Al-Oxiden basierenden Katalysatoren sowie durch Einhalten bestimmter Reaktionsbedingungen erreicht. Typische, mit diesem Verfahren erhaltene Mischungen enthalten ca. 15 bis 20 Mol-% 1,4-Butandiol und 60 bis 80 Mol-% THF, wobei die Menge an THF sogar entsprechend einem Beispiel auf über 99 Mol-% gesteigert werden kann. Dies wird dadurch erreicht, daß GBL als Lösungsmittel eingesetzt wird, und zwar in einem mehrfachen Überschuß. Wird dagegen ohne Lösungsmittel gearbeitet, sinken die Ausbeuten beträchtlich auf Werte von 75 %.

Die EP-A 0 404 408 dagegen offenbart einen Katalysator für die MSA-Hydrierung, dessen Aufbau prinzipiell anders ist als derjenige der Katalysatoren in den vorstehend genannten Referenzen. Das katalytisch aktive Material entspricht dabei im wesentlichen dem Material, das in der oben zitierten US 5,072,009 offenbart ist. Das Material wird dann auf einem im wesentlichen inerten, mindestens teilweise porösen, eine äußere Oberfläche aufweisenden Träger aufgetragen. Das katalytisch aktive Material haftet an der äußeren Oberfläche des Trägers. Im Gegensatz zu dem entsprechenden, nicht auf einem Träger angebrachten Katalysator, der als Hauptprodukt THF liefert, entsteht hierbei GBL als bevorzugtes Produkt. Auch hierbei enthalten sämtliche in den Beispielen verwendeten Katalysatoren Cr. Nachteilig ist hierbei auch die große Menge an gebildetem BSA.

Alle die in den vorstehend genannten Druckschriften beschriebenen Katalysatortypen weisen den Nachteil auf, daß sie noch eine große Menge an unerwünschtem Nebenprodukt liefern bzw. nur für die Herstellung eines der prinzipiell erwünschten Hauptprodukte THF und GBL einsetzbar sind. Häufig enthalten die Katalysatoren auch Cr.

Ein zweistufiges Verfahren zur Hydrierung von MSA ist in der Patentschrift US, 5,149,836 beschrieben. Mit diesem Verfahren können GBL und THF mit einem einstellbaren Selektivitätsverhältnis von 15 bis 92% GBL bzw. 7 bis 83% THF produziert werden. Das Verfahren umfaßt eine erste Stufe, in der MSA an einem ersten Katalysatorbett aus 30 bis 65 Gew.-% CuO, 18 bis 50 Gew.-% ZnO und 8 bis 22 Gew.-% Al₂O₃ zu einem überwiegend GBL enthaltenden Gasgemisch hydriert wird. An einem zweiten Katalysatorbett, bestehend aus 10 bis 50 Gew.-% CuO, 30 bis 65 Gew.-% ZnO und 3 bis 20 Gew.-% Cr₂O₃ wird das in der ersten Stufe erhaltende GBL zu THF hydriert. Die Temperaturen der ersten Hydrierung liegen zwischen 200 und 400°C, die der zweiten zwischen 200 bis 350°C, vorzugsweise 250 bis 280°C. Nach den Beispielen liegt die Reaktionstemperatur in der ersten Stufe bei Werten von 245 bis 275°C, die der zweiten Stufe bei Werten von 250 bis 280°C. Bei einer Temperatur von 250°C wird dabei in der zweiten Hydrierstufe hauptsächlich Butandiol gebildet, wogegen bei 280°C hauptsächlich THF entsteht.

In WO 99/35136 wird ein weiteres Verfahren zur Herstellung von THF und GBL in wechselnden relativen Mengenverhältnissen beschrieben. Als Edukte dienen Maleinsäureoder Bemsteinsäureanhydrid oder Fumarsäureester. Diese werden in einer ersten Stufe an einem kupferbasierenden Heterogenkatalysator mit Wasserstoff umgesetzt, vorzugsweise werden Kupfer-Zinkoxid- oder stabilisierte Kupferchromit-Katalysatoren eingesetzt. In einer zweiten Reaktionsstufe kommt ein saures Silicium-Aluminiumoxid zum Einsatz.. Nachteilig an diesem Verfahren ist neben der Verwendung von zwei völlig unterschiedlichen Katalysatoren auch die nur eingeschränkte Flexibilität bezüglich des Produktmixes, da das Verhältnis GBL : THF nur im Bereich von 70 : 30 bis 40 : 60 variiert werden kann.

Es ist die Aufgabe der vorliegenden Erfindung, ein Verfahren der Gasphasenhydrierung von Maleinsäure und/oder Bernsteinsäure und/oder den oben erwähnten Derivaten zur Verfügung zu stellen, mit dem gegebenenfalls substituiertes GBL und/oder THF herstellbar ist und das es gestattet, diese beiden Produkte in stark variierenden relativen Mengenverhältnissen zueinander und in hohen Ausbeuten herzustellen.

Diese Aufgabe wird gelöst durch ein Verfahren der Gasphasenhydrierung von C₄-Dicarbonsäuren und/oder deren Derivaten zu gegebenenfalls substituiertem γ-Butyrolacton und/oder Tetrahydrofuran an einem Katalysator auf Cu-Oxid-Basis, umfassend eine erste Hydrierstufe, in der die C₄-Dicarbonsäure oder deren Derivat zu einem gegebenenfalls substituiertes γ-Butyrolacton als Hauptprodukt enthaltenden Gemisch umgesetzt wird, und eine sich daran anschließende zweite Hydrierstufe, in der das im Gemisch aus der ersten Hydrierstufe befindliche, gegebenenfalls substituierte γ-Butyrolacton bei einer Temperatur, die niedriger ist als die Temperatur in der ersten Hydrierstufe, zu gegebenenfalls substituiertem Tetrahydrofuran umgesetzt wird.

Unter dem Begriff C₄-Dicarbonsäuren und deren Derivate werden im Bezug auf die vorliegende Anmeldung verstanden Maleinsäure und Bernsteinsäure, die gegebenenfalls einen oder mehrere C₁-C₆-Alkylsubstituenten aufweisen sowie die Anhydride und Ester dieser gegebenenfalls alkylsubstituierten Säuren. Ein Beispiel einer solchen Säure ist Citraconsäure. Vorzugsweise wird MSA eingesetzt.

Es wurde überraschend gefunden, daß durch den Einsatz der Cr-freien Hydrierkatalysatoren, die hintereinandergeschaltet sind, sowie das Einhalten bestimmter Reaktionsbedingungen das Produktverhältnis von GBL : THF in weiten Grenzen variiert werden kann.

In beiden Reaktionszonen wird dabei ein Cr-freier Katalysator auf der Basis von Cu-Oxid verwendet. Dieses ist in Mengen von 5 bis 100 Gew.-% vorhanden. Der Katalysator kann weiterhin ein oder mehrere Metalle oder eine Verbindung davon, vorzugsweise ein Oxid, aus der Gruppe bestehend aus Al, Si, Zn, La, Ce, den Elementen der Gruppe IIIA bis VIIIA sowie den Gruppen IA und IIA, in Mengen von 0 bis 95 Gew.-% aufweisen. Die Katalysatoren, die in den beiden Reaktionszonen verwendet werden, können dabei identisch sein oder unterschiedliche Zusammensetzungen aufweisen. Welche dieser beiden Ausführungsformen gewählt wird, richtet sich beispielsweise nach der gewünschten Produktzusammensetzung.

Die Gruppe des Periodensystems der Elemente werden im Zusammenhang mit der vorliegenden Erfindung nach der alten IUPAC-Nomenklaatur bezeichnet.

Die in der ersten Hydrierstufe verwendeten Katalysatoren enthalten vorzugsweise 5 bis 100 Gew.-% CuO, 0 bis 80 Gew.-% ZnO und 0 bis 95 Gew.-% Al₂O₃, insbesondere 20 bis 80 Gew.-% CuO, 10 bis 40 Gew.-% ZnO und 5 bis 60 Gew.-% Al₂O₃. Die in der zweiten Hydrierstufe benutzen Katalysatoren enthalten vorzugsweise 5 bis 80 Gew.-% CuO, 0 bis 80 Gew.-% ZnO und 0 bis 60 Gew.-% Al₂O₃, insbesondere 20 bis 60 Gew.-% CuO, 0 bis 60 Gew.-% ZnO und 10 bis 50 Gew.-% Al₂O₃.

Das gemischte, erfindungsgemäß verwendete Oxid kann auch die jeweiligen Metalle in elementarer Form enthalten. Diese entstehen insbesondere unter einer reduzierenden Wasserstoffatmopshäre. Generell wird der Katalysator vor dem Einsatz in die Reaktion einer Aktivierung, im allgemeinen einer Wasserstoffvorbehandlung, unterzogen. Dadurch wird die aktive Katalysatorspezies hergestellt. Dies geschieht durch ein teilweises Reduzieren der in der Katalysatormischung vorhandenen Oxide zum elementaren Metall, das in der erfindungsgemäßen katalytischen Reaktion aktiv ist.

Vorzugsweise wird in der ersten Hydrierstufe eine Temperatur von ≥200°C, insbesondere 230 bis 300°C, eingehalten. Als Primärprodukt dieser ersten Hydrierung entsteht gegebenenfalls substituiertes Bernsteinsäureanhydrid (BSA), das dann weiter zu gegebenenfalls substituiertem GBL hydriert wird. Bei zu niedriger Temperatur ist daher eine Desaktivierung des Katalysators durch Belegung mit schwerflüchtigem BSA zu beobachten, wenn die Hydrierreaktion unterhalb von 200°C durchgeführt wird.

Die Reaktion wird so durchgeführt, daß in der ersten Reaktionszone das Eduktgemisch mit Wasserstoff zu einem Produktgemisch umgesetzt wird, das überwiegend gegebenenfalls substituiertes GBL enthält. Hierfür wird das Eduktgemisch verdampft und mit einem Wasserstoff enthaltenden Gasstrom durch den Reaktor geleitet. Dabei wird versucht, den Wasserstoffanteil in dem Gasstrom möglichst hoch einzustellen. Es können andere gasförmige Komponenten, wie Wasserdampf, Kohlenwasserstoffe wie Methan, Ethan oder n-Butan, oder Kohlenmonoxid, vorhanden sein. Die Reaktionsbedingungen (Temperatur, Druck, GHSV, MSA-Eingangskonzentration) sowie der Katalysator werden so gewählt, daß die GBL-Ausbeute maximal wird, während die Bildung von BSA oder Überhydrierprodukten in untergeordnetem Maß eintritt. Es werden dabei GBL-Ausbeuten von mindestens etwa 30 % angestrebt. Bevorzugt sind GBL-Ausbeuten von mindestens etwa 50 %, insbesondere mindestens etwa 70 %. Zu hohe Temperaturen begünstigen die Bildung von unerwünschten Nebenprodukten.

Die Konzentration der Edukts liegt zwischen 0,1 und 5 Vol.-%, bevorzugt zwischen 0,2 und 3 Vol.-%. Bei wesentlich höheren Konzentrationen kondensiert das Edukt im Reaktor aus und belegt den Katalysator mit einem Flüssigkeitsfilm. Dies ist vor allem bei MSA zu beobachten. Wesentlich geringere Konzentrationen würden die Raum-Zeit-Ausbeute verringern und das Verfahren unnötig verteuern. Die GHSV (Gas Hourly Space Velocity = Volumenstrom des Reaktionsgases bei Normbedingungen bezogen auf das Katalysatorschüttvolumen) wird so eingestellt, daß die Edukte und das BSA vollständig umgesetzt werden, vorzugsweise auf Werte von 100 bis 10.000 h⁻¹. Der Druck liegt bei Werten von 0,5 bis 100 bar, vorzugsweise von 1 bis 50 bar, insbesondere < 20 bar. Höhere Drücke erleichtern zwar die Umsetzung der Edukte, verteuern jedoch auch die Kosten des Verfahrens. Als Reaktor eignen sich Rohrreaktoren, Rohrbündelreaktoren, in denen der Katalysator als feste Schüttung angeordnet ist, und Wirbelschichtreaktoren.

In der zweiten Reaktionszone wird eine Temperatur eingestellt, die unterhalb derjenigen in der vorherigen Hydrierstufe liegt. Vorzugsweise wird die zweite Hydrierung bei Temperaturen ≤ 280°C, vorzugsweise 150 bis 240°C, durchgeführt. Zu hohe Temperaturen führen zur Bildung von Nebenprodukten durch Überhydrierung und somit zu Ausbeuteminderung. Generell wird so vorgegangen, daß das Reaktorabgas der ersten Reaktionszone anschließend in die zweite Reaktionszone geleitet wird, vorzugsweise ohne weitere Aufarbeitung. Da die Temperatur der zweiten Stufe niedriger als die der ersten ist, sollte das Reaktionsgas auf die Temperatur der zweiten Stufe abgekühlt werden. Als Reaktor für die zweite Stufe eignen sich ebenfalls Rohrreaktoren, Rohrbündelreaktoren oder Wirbelschichtreaktoren. Die Reaktionsbedingungen (Temperatur, Druck, GHSV) sowie der Katalysator in der zweiten Reaktionszone werden so ausgewählt, daß sich GBL entsprechend dem gewünschten Selektivitätsverhältnis zu gegebenenfalls substituiertem THF umsetzt. Zu tiefe Temperaturen führen zu einem unnötigen Verlust an Raum-Zeit-Ausbeute des Katalysators. Für die GHSV sowie für den Druck gelten die gleichen Bereiche wie im Fall der ersten Reaktionszone. Die Zusammensetzung des Reaktionsgases bei Eintritt in die zweite Stufe ist von den Bedingungen in der ersten Stufe abhängig. Entsprechend liegt die GBL-Konzentration zwischen vorzugsweise 0,2 und 2,0 Vol.-%. Das Produktgemisch kann nach den dem Fachmann bekannten Verfahren getrennt werden; der überschüssige Wasserstoff kann in Kreisgasfahrweise erneut zur Hydrierung eingesetzt werden.

Gemäß einer Variante der Erfindung sind für beide Reaktionszonen in einem Reaktor angebracht. Geeignet ist ein Rohrreaktor, ein Rohrbündelreaktor oder eine Kombination aus diesen. Zur Einstellung der bevorzugten Temperaturen in den beiden Reaktionszonen können ein oder mehrere Heizkreisläufe verwendet werden. Die hydrierende Umsetzung von MSA, Maleinsäure oder ihrer Ester zu GBL und THF ist mit einer großen Wärmefreisetzung verbunden. Hierbei ist die Reaktionsenthalpie der Reaktion zu GBL höher als die der Hydrierung von GBL zu THF. In einem nicht isotherm betriebenen Reaktor wird daher die Temperatur im vorderen Teil des Reaktors höher als im hinteren sein. Es ist daher besonders bevorzugt, durch dem Fachmann bekannte Maßnahmen ein Temperaturprofil entlang der Reaktorlängsachse zu erzeugen, so daß sich die bevorzugten Temperaturen der beiden Reaktionszonen im Reaktor einstellen. Die Form des Temperaturprofils hängt von dem Fachmann bekannten Parametern, beispielsweise der auf das Volumen bezogenen Katalysatoraktivität, von den Reaktionsbedingungen (Druck, GHSV und Eingangskonzentration der Edukte) sowie von der Geometrie und Thermostatisierung des Reaktors ab.

Das erfindungsgemäße Verfahren erlaubt (GBL + THF)-Ausbeuten, die bei ≥ 98% liegen. Das Verhältnis GBL/THF läßt sich in Bereichen von ca. 90 : 10 bis 0 : 100 variieren.

### Beispiel

In der ersten Reaktionszone wurden 100 ml eines Katalysators der Zusammensetzung 70 Gew.-% CuO, 25 Gew.-% ZnO und 5 Gew.-% Al₂O₃ mit 100 ml Glasringen der gleichen Größe gemischt und in einen Rohrreaktor gefüllt. Der Reaktor war temperiert und wurde von oben nach unten mit dem Reaktionsgas durchströmt. MSA wurde als Schmelze in einen bei 200°C betriebenen Verdampfer gepumpt, wo es in einem Wasserstoffstrom verdampft wurde. Das MSA-Wasserstoff-Gasgemisch mit einer MSA-Konzentration von 1,0 Vol.-% wurde dann durch den Reaktor geleitet. Um das Gas auf die Reaktionstemperatur vorzuheizen, wurde oberhalb der Katalysatorschüttung eine Schüttung aus 100 ml Glasringen eingefüllt.

Vor dem Einspeisen des MSA-Wasserstoffgemisches wurde der Katalysator einer Wasserstoff-vorbehandlung unterzogen. Dazu wurde zunächst der Reaktor mit 200 Nl/h Stickstoff bei Atmosphärendruck gespült und gleichzeitig innerhalb einer Stunde auf eine Temperatur in der Katalysatorschüttung von 180°C aufgeheizt. Danach wurde der Stickstoffvolumenstrom auf 950 Nl/h erhöht und zusätzlich 50 Nl/h Wasserstoff eingespeist. Dabei wurde eine leichte Temperaturerhöhung in der Katalysatorschüttung auf etwa 250°C beobachtet. Nachdem die Temperatur in der gesamten Katalysatorschüttung auf 190°C abgekühlt war, wurde der Stickstoffvolumenstrom allmählich auf 500 Nl/h erniedrigt und der Wasserstoffstrom auf 500 Nl/h erhöht. Schließlich wurde der Stickstoffvolumenstrom abgeschaltet und der Wasserstoffstrom auf 600 Nl/h angehoben.

Die Reaktion wurde bei 5 bar Druck und 240°C durchgeführt. Die GHSV betrug 3000h⁻¹.

Bei vollständigem MSA-Umsatz wurde BSA im Reaktorabgas nicht nachgewiesen. Die Selektivitäten zu GBL und THF betrugen 88 bzw. 10%. Überhydrierprodukte (hauptsächlich Butanol und Butan) wurden mit 2% Selektivität gebildet.

Die Reaktionsabgase wurden dann in die zweite Reaktionszone geleitet. Diese wurde hergestellt, indem 100 ml eines Katalysators der Zusammensetzung 40 Gew.-% CuO, 40 Gew.-% ZnO und 20 Gew.-% Al₂O₃ mit 100 ml Glasringen der gleichen Größe gemischt und in einen Rohrreaktor gefüllt wurden. Vor Durchführung der Reaktion wurde der Katalysator nach der oben beschriebenen Wasserstoffvorbehandlung formiert. Die Reaktionsabgase wurden zur Temperatureinstellung vor dem Einspeisen in die zweite Reaktionszone in einem bei 150°C betriebenen Verdampfer mit einem Wasserstoffstrom gemischt. Das GBL-THF-Wasserstoff-Gasgemisch mit einer Zusammensetzung von 1,0 Vol.-% GBL und 0,1 Vol.-% THF wurde dann durch den Reaktor geleitet. Die Reaktion erfolgte bei 190°C und 5 bar. Die GHSV betrug 3000 h⁻¹.

Sämtliches im Reaktionsabgas befindliches GBL wurde vollständig umgesetzt. Die Ausbeute an THF bezogen auf das eingesetzte GBL betrug > 99%. Es wurde keine Nebenproduktbildung beobachtet.

Durch Erniedrigung der Temperatur kann der GBL-Umsatz herabgesetzt werden, wodurch das Reaktionsgas mehr GBL und weniger THF beinhaltet Im Extremfall kann die Temperatur so weit gesenkt werden, daß keine Umsetzung des GBL erfolgt.

## Patentansprüche

1. Verfahren der Gasphasenhydrierung von C₄-Dicarbonsäuren und/oder deren Derivaten zu gegebenenfalls substituiertem γ-Butyrolacton und/oder Tetrahydrofuran an einem Katalysator auf Cu-Oxid-Basis, umfassend eine erste Reaktionszone, in der die C₄-Dicarbonsäure und/oder deren Derivate zu einem gegebenenfalls substituiertes γ-Butyrolacton als Hauptprodukt enthaltenden Gemisch umgesetzt wird, und eine sich daran anschließende zweite Reaktionszone, in der das im Gemisch aus der ersten Hydrierstufe befindliche gegebenenfalls substituierte γ-Butyrolacton bei einer Temperatur, die niedriger ist als die Temperatur in der ersten Hydrierstufe, zu gegebenenfalls substituiertem Tetrahydrofuran umgesetzt wird.

2. Verfahren nach Anspruch 1, **dadurch gekennzeichnet, daß** der Katalysator 5 bis 100 Gew.-% Cu-Oxid und 0 bis 95 Gew.-% eines oder mehrerer Metalle oder deren Verbindungen, vorzugsweise deren Oxid, aus der Gruppe bestehend aus Al, Si, Zn, La, Ce, den Elementen der Gruppe IIIA bis VIIIA sowie der Gruppe IA und IIA als aktive Masse aufweist.

3. Verfahren nach Anspruch 1 oder 2, **dadurch gekennzeichnet, daß** die Reaktion in der ersten Zone bei Temperaturen von ≥ 200°C, vorzugsweise 230 bis 300°C, und die Reaktion der zweiten Zone bei Temperaturen von ≤ 280°C, vorzugsweise 150 bis 240°C, durchgeführt wird.

4. Verfahren nach einem der Ansprüche 1 bis 3, **dadurch gekennzeichnet, daß** die in den beiden Reaktionszonen verwendeten Katalysatoren die gleiche Zusammensetzung aufweisen.

5. Verfahren nach einem der Ansprüche 1 bis 3, **dadurch gekennzeichnet, daß** die in den beiden Reaktionszonen verwendeten Katalysatoren unterschiedliche Zusammensetzungen aufweisen.

6. Verfahren nach einem der Ansprüche 1 bis 5, **dadurch gekennzeichnet, daß** der in der ersten Reaktionszone verwendete Katalysator von 5 bis 100 Gew.-% CuO, 0 bis 80 Gew.-% ZnO und 0 bis 95 Gew.-% Al₂O₃, insbesondere 20 bis 80 Gew.-% CuO, 10 bis 40 Gew.-% ZnO und 5 bis 60 Gew.-% Al₂O₃ und der in der zweiten Reaktionszone verwendete Katalysator von 5 bis 80 Gew.-% CuO, 0 bis 80 Gew.-% ZnO und 0 bis 60 Oew.-% Al₂O₃, insbesondere 20 bis 60 Gew.-% CuO, 0 bis 60 Gew.-% ZnO und 10 bis 50 Gew.-% Al₂O₃ enthält.

7. Verfahren nach einem der Ansprüche 1 bis 6, **dadurch gekennzeichnet, daß** für beide Reaktionszonen ein nicht isotherm betriebener Reaktor verwendet wird, der so eingestellt wird, daß die Temperatur in der ersten Reaktionszone höher ist als in der zweiten Reaktionszone.

8. Verfahren nach einem der Ansprüche 1 bis 7, **dadurch gekennzeichnet, daß** die in beiden Reaktionszonen eingestellte Druck unabhängig voneinander bei Werten von 0,5 bis 100 bar, vorzugsweise 1 bis 50 bar, insbesondere < 20 bar, liegt.

9. Verfahren nach einem der Ansprüche 1 bis 8, **dadurch gekennzeichnet, daß** Maleinsäureanhydrid als Edukt eingesetzt wird.

10. Verfahren nach Anspruch 9, **dadurch gekennzeichnet, daß** die Maleinsäureanhydrid-Konzentration in der ersten Reaktionsstufe bei Werten von 0,1 bis 5 Vol.-%, vorzugsweise 0,2 bis 3 Vol.-%, liegt.

11. Hydrierverfahren nach einem der Ansprüche 1 bis 10, **dadurch gekennzeichnet, daß** die erste und zweite Reaktion unabhängig voneinander in einem Rohrreaktor, einem Rohrbündelreaktor oder einem Wirbelschichtreaktor durchgeführt werden.

## Claims

1. A process for the gas-phase hydrogenation of C₄-dicarboxylic acids and/or their derivatives over a catalyst based on copper oxide to give substituted or unsubstituted γ-butyrolactone and/or tetrahydrofuran, which comprises a first reaction zone in which the C₄-dicarboxylic acid and/or its derivatives is/are reacted to give a mixture comprising a substituted or unsubstituted γ-butyrolactone as main product and a subsequent second reaction zone in which the substituted or unsubstituted γ-butyrolactone present in the mixture from the first hydrogenation step is reacted at a temperature lower than the temperature in the first hydrogenation step to give substituted or unsubstituted tetrahydrofuran.

2. A process as claimed in claim 1, wherein the catalyst comprises from 5 to 100% by weight of copper oxide and from 0 to 95% by weight of one or more metals or their compounds, preferably their oxides, selected from the group consisting of Al, Si, Zn, La, Ce, the elements of groups IIIA to VIIIA and groups IA and IIA as active composition.

3. A process as claimed in claim 1 or 2, wherein the reaction in the first zone is carried out at ≥ 200°C, preferably from 230 to 300°C, and the reaction in the second zone is carried out at ≤ 280°C, preferably from 150 to 240°C.

4. A process as claimed in any of claims 1 to 3, wherein the catalysts used in the two reaction zones have the same composition.

5. A process as claimed in any of claims 1 to 3, wherein the catalysts used in the two reaction zones have different compositions.

6. A process as claimed in any of claims 1 to 5, wherein the catalyst used in the first reaction zone comprises from 5 to 100% by weight of CuO, from 0 to 80% by weight of ZnO and from 0 to 95% by weight of Al₂O₃, in particular from 20 to 80% by weight of CuO, from 10 to 40% by weight of ZnO and from 5 to 60% by weight of Al₂O₃, and the catalyst used in the second reaction zone comprises from 5 to 80% by weight of CuO, from 0 to 80% by weight of ZnO and from 0 to 60% by weight of Al₂O₃, in particular from 20 to 60% by weight of CuO, from 0 to 60% by weight of ZnO and from 10 to 50% by weight of Al₂O₃.

7. A process as claimed in any of claims 1 to 6, wherein both reaction zones are accommodated in a reactor which is not operated isothermally and in which the temperatures are set so that the temperature in the first reaction zone is higher than that in the second reaction zone.

8. A process as claimed in any of claims 1 to 7, wherein the pressures set in the two reaction zones are, independently of one another, from 0.5 to 100 bar, preferably from 1 to 50 bar, in particular < 20 bar.

9. A process as claimed in any of claims 1 to 8, wherein maleic anhydride is used as starting material.

10. A process as claimed in claim 9, wherein the maleic anhydride concentration in the first reaction step is from 0.1 to 5% by volume, preferably from 0.2 to 3% by volume.

11. A hydrogenation process as claimed in any of claims 1 to 10, wherein the first and second reactions are carried out, independently of one another, in a tube reactor, a shell-and-tube reactor or a fluidized-bed reactor.

## Revendications

1. Procédé d'hydrogénation en phase gazeuse d'acides dicarboxyliques en C₄ et/ou de leurs dérives en γ-butyrolactone et/ou tétrahydrofuranne le cas échéant substitués à un catalyseur à base d'oxyde de cuivre, comprenant une première zone réactionnelle, dans laquelle l'acide dicarboxylique en C₄ et/ou le dérivé de celui-ci est transformé en un mélange contenant une γ-butyrolactone substituée le cas échéant comme produit principal, et une seconde zone réactionnelle suivant celle-ci, dans laquelle la γ-butyrolactone substituée le cas échéant se trouvant dans le mélange de la première étape d'hydrogénation est transformée en tétrahydrofuranne substitué le cas échéant à une température qui est inférieure à la température dans la première étape d'hydrogénation.

2. Procédé selon la revendication 1, **caractérisé en ce que** le catalyseur présente 5 à 100 % en poids d'oxyde de cuivre et 0 à 95 % en poids d'un ou plusieurs métaux ou des composés de ceux-ci, de préférence des oxydes de ceux-ci, du groupe comprenant Al, Si, Zn, La, Ce, les éléments des groupes IIIA à VIIIA ainsi que du groupe IA et IIA en tant que masse active.

3. Procédé selon la revendication 1 ou 2, **caractérisé en ce que** la réaction dans la première zone réactionnelle est effectuée à des températures de ≥ 200°C, de préférence 230 à 300°C, et la réaction de la seconde zone réactionnelle est effectuée à des températures de ≤ 280°C, de préférence 150 à 240°C.

4. Procédé selon l'une des revendications 1 à 3, **caractérisé en ce que** les catalyseurs utilisés dans les deux zones réactionnelles présentent la même composition.

5. Procédé selon l'une des revendications 1 à 3, **caractérisé en ce que** les catalyseurs utilisés dans les deux zones réactionnelles présentent des compositions différentes.

6. Procédé selon l'une des revendications 1 à 5, **caractérisé en ce que** le catalyseur utilisé dans la première zone réactionnelle contient de 5 à 100 % en poids de CuO, 0 à 80 % en poids de ZnO et 0 à 95 % en poids de Al₂O₃, en particulier 20 à 80 % en poids de CuO, 10 à 40 % en poids de ZnO et 5 à 60 % en poids de Al₂O₃ et le catalyseur utilisé dans la seconde zone réactionnelle contient de 5 à 80 % en poids de CuO, 0 à 80 % en poids de ZnO et 0 à 60 % en poids de Al₂O₃, en particulier 20 à 60 % en poids de CuO, 0 à 60 % en poids de ZnO et 10 à 50 % en poids de Al₂O₃.

7. Procédé selon l'une des revendications 1 à 6, **caractérisé en ce que** pour les deux zones réactionnelles, un réacteur fonctionnant en non isotherme est employé, qui est mis au point de telle manière que la température dans la première zone réactionnelle est plus haute que dans la seconde zone réactionnelle.

8. Procédé selon l'une des revendications 1 à 7, **caractérisé en ce que** la pression mise au point dans les deux zones réactionnelles indépendamment l'une de l'autre s'élève à des valeurs de 0,5 à 100 bars, de préférence 1 à 50 bars, en particulier < 20 bars.

9. Procédé selon l'une des revendications 1 à 8, **caractérisé en ce que** l'anhydride de l'acide maléique est employé comme éduit.

10. Procédé selon la revendication 9, **caractérisé en ce que** la concentration en anhydride de l'acide maléique s'élève dans la première étape de réaction à des valeurs de 0,1 à 5 % en volume de préférence 0,2 à 3 % en volume.

11. Procédé d'hydrogénation selon l'une des revendications 1 à 10, **caractérisé en ce que** la première et la seconde réaction indépendamment l'une de l'autre sont effectuées dans un réacteur tubulaire, un réacteur multitubulaire ou un réacteur en lit fluidisé.
